# EUROPEAN PATENT SPECIFICATION

(11) **EP 2 197 284 B1**
(45) Date of publication and mention of the grant of the patent: **12.04.2017**
(21) Application number: 08806507.3
(22) Date of filing: 03.10.2008
(51) Int. Cl.: A01N 63/02, A61K 35/74, A61K 35/76, A61K 8/99, A01P 1/00

(54) **ANTI-BACTERIAL COMPOSITIONS**
ANTIBAKTERIELLE ZUSAMMENSETZUNGEN
COMPOSITIONS ANTIBACTÉRIENNES

(30) Priority: 04.10.2007 GB 0719438; 16.01.2008 GB 0800790
(43) Date of publication of application: 23.06.2010
(73) Proprietor: AmpliPhi Biosciences Corporation, San Diego, CA 92130 (US)
(72) Inventor: JIA, Ying, Oadby, Leicestershire LE2 5TS (GB)
(74) Representative: MacLean, Martin Robert
(86) International application number: PCT/GB2008/003363
(87) International publication number: WO 2009/044163

(56) References cited:
- WO-A1-2004/004495
- WO-A1-2007/148919
- WO-A2-01/50872
- WO-A2-01/51066
- WO-A2-03/080823
- O'FLAHERTY S ET AL: "Potential of the polyvalent anti-Staphylococcus bacteriophage K for control of antibiotic-resistant staphylococci from hospitals" APPLIED AND ENVIRONMENTAL MICROBIOLOGY, AMERICAN SOCIETY FOR MICROBIOLOGY, US, vol. 71, no. 4, 1 April 2005 (2005-04-01), pages 1836-1842, XP002449939 ISSN: 0099-2240 cited in the application
- N. CERCA, R. OLIVEIRA & J. AZEREDO: "Susceptibility of Staphylococcus epidermidis planktonic cells and biofilms to the lytic action of staphylococcus bacteriophage K." LETT. APPL. MICROBIOL., vol. 45, September 2007 (2007-09), pages 313-317, XP002558222
- M. TAKAC & U. BLÄSI: "Phage P68 virion-associated protein 17 displays activity against clinical isolates of Staphylococcus aureus" ANTIMICROB. AGENTS AND CHEMOTHERAPY, vol. 49, no. 7, July 2005 (2005-07), pages 2934-2940, XP002558223 cited in the application
- SOOTHILL J S: "BACTERIOPHAGE PREVENTS DESTRUCTION OF SKIN GRAFTS BY PSEUDOMONAS AERUGINOSA" BURNS, BUTTERWORTH HEINEMANN, GB, vol. 20, no. 3, 1 January 1994 (1994-01-01), pages 209-211, XP009009002 ISSN: 0305-4179
- K. ASAMI ET AL.: "Synchronized disruption of Escherichia coli cells by T4 phage infection" J. FERMENTATION AND BIOENGINEERING, vol. 83, no. 6, 1997, pages 511-516, XP002558224

## Description

Described herein are formulations comprising bacteriophages ("phages") and methods of treating bacterial infections, or disinfecting surfaces, using high concentrations of bacteriophage and/or formulations containing phage K and/or P68. The
invention makes use of the phenomenon known as "lysis-from-without".

Antibacterial agents, in the form of chemically-based antibiotics (i.e. non-viral agents), such as penicillin or tetracycline, are well known. The problem with such antibiotics is that resistance to them is becoming increasingly common. Mutations conferring antibiotic resistance, or genes encoding antibiotic resistance enzymes, such as penicillinases, are becoming increasingly common in pathogenic bacteria world-wide. Methicillin-resistant *Staphylococcus atreus* (MRSA) bacteria, for example, are an increasingly common form of infection, often acquired during surgery for other causes at hospitals. MRSA infections are extremely difficult to treat using conventional antibiotics.

One alternative approach to treating bacterial infections is to infect the bacteria with a virus, known as a bacteriophages. Such "bacteriophage therapy" was first developed early in the twentieth century, but has been little used in the West since the advent of antibiotics in the 1940s. More extensive work has been carried out in Eastern Europe.

Bacteriophages (also known as "phages,") are specific to specific kinds of bacterial cells. They cannot infect the cells of more complex organisms because of major differences in key intracellular machinery, as well as in cell-surface components. Most bacteriophages have structures, such as tail fibres, which enable the bacteriophages to bind to specific molecules on the surface of their target bacteria. Viral DNA within the bacteriophages, or RNA in some bacteriophages, is then injected, usually through the tail, into the host cell, which then directs the production of progeny bacteriophage.

Different kinds of bacteriophages are found which infect different bacteria. Conventionally, they can be isolated from the environment in which the particular bacterium grows, for example from sewage or faeces. The presence of a bacteriophage in a sample may be determined by passing the sample through a filter with pores small enough to prevent the bacteria getting through the filter. The filtered extract is usually mixed with growth medium, suitable host bacteria added, and then spread on, for example, an agar plate. The presence of clear spots, called plaques, on the resulting lawn of bacteria indicates the presence of one or more bacteriophages, which cause the bacteria to lyse.

Bacteriophages which can only kill bacteria are known as "obligately lytic" bacteriophages. Obligately lytic bacteriophages exist outside the bacterial cell in the form of nucleic acid material, usually DNA, surrounded by a protein coat. The protein coat usually has one or more molecules attached to it which allow the bacteriophages to attach to specific molecules on the surface of the bacteria. Upon binding to the bacteria the DNA gains entry into the bacterial host where it is transcribed and translated into various proteins necessary for replication and assembly of new bacteriophage. The DNA is also replicated and is packaged into new bacteriophage which are released upon lysis of the bacterial cell.

In addition to obligately lytic bacteriophages there are lysogenic, or temperate bacteriophages. These temperate bacteriophages have two life cycles, one in which they lyse the infected cell, and the other in which they enter the prophage state. Obligately lytic bacteriophages always have to infect from outside, reprogram the host cell and release a burst of bacteriophage through breaking open or lysing the infected cell. "Temperate" bacteriophages may integrate their DNA into the host bacterial DNA leading to a virtually permanent association as a prophage within a specific bacterium and its progeny. Some prophages do not physically integrate into the chromosome, but exist as an autonomous replicon. The prophage directs the synthesis of a repressor which blocks the expression of its own genes and also those of any closely-related temperate bacteriophages. Occasionally, the prophage may escape regulation by its repressor. The prophage DNA may then be cut out of the genome by site-specific recombination, replicated, and the progeny released from the host cell, in most cases by lysis.

Obligately lytic bacteriophage have been used to treat bacterial infections. Isolated obligately lytic bacteriophages have been applied to wounds or injected intravenously where they kill bacteria. The advantage of bacteriophages is that they are self-replicating, with as few as one hundred or so bacteriophages being able to kill as many as one hundred million bacteria. The bacteriophages simply replicate themselves by killing bacteria until they have eliminated them from the individual or the environment. WO 01/51066, for example, discloses a method of treating a patient with one or more obligately lytic bacteriophages. Similarly, US 4,957,686 discloses a method of treating dental caries with bacteriophage.

One possible problem with using bacteriophages has been that the patient's own body will often have an immune response against the bacteriophages and eliminate the bacteriophages from blood. US 5,660,812, US 5,688,501 and US 5,766,892 all show methods of selecting bacteriophages to improve the bacteriophage half-life within the blood of a patient to be treated. US 5,811,093 discusses selecting a modified gene encoding one of the capsid (coating) proteins (capsid E) so that the bacteriophages survive in an animal's circulatory system for longer. In the case of the latter patent, the modification was identified as a point mutation within a gene.

WO 01/50872 A2 discloses a method for sanitation of produce using bacteriophages.

Soothill (1999) Burns, Vol. 20, No. 3, 209-211 discloses that infection of split skin grafts in guinea-pigs by *Pseudomonas aeruginosa* 3719 destroys them, and bacteriophage BS24, lytic for strain 3719, protects the grafts.

A problem associated with prior art uses to disinfect or treat bacterial contaminants or diseases is that phage are often bacterial strain specific. The presence of, for example, a prophage within a bacterium may block the expression of genes from an infectious phage, thus preventing replication of the infectious phage and preventing lysis and killing of the bacterium. A prophage may also cause the destruction of incoming phage DNA.

This has previously meant that either the phage needs to be matched to the bacterium, often requiring complicated genetic analysis of the bacterium, or a number of different phages need to be used in combination. The production of panels of different phages, such as panels of *vir* mutants derived from temperate bacteriophage, is disclosed in WO 03/080823.

The phenomenon known as "lysis from without" has been known since the late 1930's and early 1940's. Delbrück M. (J. Gen. Physiol., (1940), pages 643-660) discusses two concepts: "lysis-from-within" and "lysis-from-without".

"Lysis-from-within" is where the bacterial cells become infected with phage, the phage multiply within the cells, and the cells lyse, releasing new phage and killing the bacteria. This is the process normally used by obligately lytic phage. "Lysis-from-without", on the other hand, does not involve replication of phage within bacterial cells. Delbrück noted that phages were adsorbed by bacterial cells, but no progeny phages were liberated. This resulted in deformation of bacterial cells into spherical bodies and killing of the bacteria by lysis. It
was noted that high concentrations of phage above a threshold value were required to induce this phenomenon.

We now know that this is due, at least in part, to digestion of the bacterial cell wall by enzymes present either in soluble form or attached to phage particles (Stent. G.S., Molecular Biology of Bacterial Particles, W.H. Freeman & Co. (1963), pages 71-87). A large amount of work has been carried out into isolating and characterising the lysis enzymes known as "lysins". Lysins are also known as murein hydrolases.

Ralston D.J., et al. (J. Gen. Physiol. (1957), 41(2): 343-358), for example, studied the action of phage and virolysin on bacteria. The virolysin had been obtained from lysates of phage-infected cells. The same group continued to report on the phenomenon. In 1964 they reported that lysis-from-without appeared to require sensitisation by phage followed by digestion of the wall by lysin (Ralston D., et al, J. Bacteriol. (1964), 88: 676-681).

The use of isolated lysins has been focussed on by the scientific community as having potential therapeutic applications. Other isolated lysis-associated enzymes and proteins, such as holins, have also been investigated. Holins are involved in the permeabilisation of cell membranes.

For example, Nelson D., et al. (PNAS, (2001), 98: 4107-4112) discloses the use of purified lysins to treat bacterial infections. The group were able to treat streptococcal infections in the upper respiratory tract using orally introduced, purified lysin. WO 01/19391 and US 2002/0094319 also disclose the use of purified obligately lytic enzymes such as lysin.

Methicillin-resistant *Staplylococcus aureus* (MRSA) can cause systemic infections or abscesses and ulcers, especially in sick, elderly or immune-compromised patients. It is increasingly a major cause of, or contribution to, death in hospitals. MRSA may reside in the nasal cavity of doctors or visitors without any apparent disease symptoms. However, the bacteria may be spread from person to person, including to patients. Accordingly, killing the bacteria assists in the prevention of the disease.

One possible approach is given in WO 03/080823, using a panel of bacteriophage. However, there are many different strains of MRSA. Hence, the panel may not kill all of the MRSA by obligately lytic infection of the bacteria. The inventor has now realised that providing phage in high enough concentrations to induce lysis-from-without provides a way of rapidly killing bacteria without having to infect the bacteria to cause lysis-from-within. This extends the number of strains that may be treated with phage, whilst still allowing lysis-from-within in susceptible strains of phage.

Other bacteria, such as *Clostridium difficile (C difficile*), are also becoming problematic in hospitals and are spread by contact with other patients, health workers or visitors, or from the surrounding environment.

Hospitals currently utilise alcoholic hand-washes to help prevent MRSA being transmitted. However, such alcoholic washes are often not suitable for use on the sensitive lining of the nasal cavity or broken areas of skin. There is therefore a need to produce a composition suitable for killing bacteria, such as MRSA or *C. difficile.*

The inventors have unexpectedly identified that applying lysis-from-without allows a simpler, cost-effective formulation to be produced. Lysis-from-without provides a way of rapidly killing bacteria. It allows the killing of antibiotic-resistant bacteria without the need for antibiotics or harmful or irritating chemicals. Moreover, utilising whole phage has the advantage that, if the phage is able to infect bacteria, via lysis-from-within, then the formulations may have a dual mode of attack; both killing by lysis-from-without and, even if the concentration of the formulation becomes diluted by bodily fluids such as mucus, by lysis-from-within.

Moreover, the phages used by the inventor, phage K and phage P68 together have been found to have activity against a wide range of bacterial strains and have additional benefits described below.

Phage K has been previously characterised as an anti-MRSA phage. O'Flaherty S.O., et al., (Appl. Environ. Microbiol. (2005), 71(4): 1836-1842) studied phage K on different drug-resistant strains of *S. aureus.* Phage P68 was studied, for example, by Takac M. and Blasi U. (Antimicrob. Agents and Chemo, (2005): 2934-2940). The inventors have found that the spectrum of MRSA strains that phage K and/or P68 infect complement each other, making the combination of the two strains especially suitable for use in anti-MRSA combinations.

Phage K has an additional advantage. Whilst the walls of Gram negative bacteria are composed primarily of peptidoglycan, Gram positive bacteria contain, in addition, large amounts of teichoic acid, an anionic polyol phosphate polymer. It has been known since the 1970s that *S*-*aureus* mutants that are resistant to phage K lack ribitol teichoic acid. (Sh aw D.R.D. et al (1970) J. Biol. Chem. 245 (19) 5101-5106). Phage K binds to bacterial cells via teichoic acid. The lack of wall teichoic acids has been found to reduce interactions with endothelial cells, teichoic acid being needed for attachment to nasal cells (Weidenmaier C et al In. J. Medical Microbiol (2008) 298, 505-513). Hence if teichoic acid is present, phage K should bind to the bacterium. If the bacterium mutates to be resistant to phage K by reducing teichoic acid in the cell wall, then this should reduce the ability of the bacterium to bind nasal cells. This should assist in reducing the virulence of any bacteria remaining after treatment with phage K.

Experimentation by the inventors has also found that, for example, phage K and its host range change derivatives at high concentrations, can still kill *S-aureus*, even if they mutate to be resistant to obligately lytic infection.

A first aspect of the invention provides a method of killing bacteria on a surface being medical equipment, bedding, furniture, walls or floors in a hospital comprising applying to the surface a disinfectant composition comprising a carrier and two or more phages, the phages comprising phage K or mutants thereof and phage P68 or mutants thereof, characterised in that the concentration of both phages is at least 5:1 pfu phage:cfu bacteria so as to induce lysis-from-without of bacteria for which the phage are a pathogen when said bacteria are present on the surface to be disinfected.

The concentration of phages needed to cause lysis-from-without may be experimentally determined, for example, by incubating bacterial cells at their normal growth temperature with different concentrations of the phage and observing at which concentrations of phages the bacterial cells lyse as shown by the culture loosing turbidity.

The phage in the composition would normally be from a predetermined particular species of bacterium. The term "a pathogen", as used herein, is intended to mean that the phage is capable of specifically binding to the surface of a species of bacterium and, when at a high concentration, capable of inducing lysis-from-without. The phage need not be capable of inducing lysis-from-within. As indicated above, the presence of different prophages within different strains of the same species of bacterium means that a particular phage may or may not induce lysis of the bacterium via the lysis-from-within mechanism. However, the phage may still bind to the surface of the bacterium and still induce lysis via the lysis-from-without mechanism.

Preferably, the concentration of the phage is at least 6:1, 7:1, 8:1, 9:1, 10:1, at least 20: 1, 40: 1, 50: 1, at least 100: 1 plaque-forming units of phage (pfu):colony forming units (cfu) of bacteria. The plaque-forming units of phage are determined on bacteria which are capable of being lysed by the phage via lysis-from-within.

It will be appreciated that the efficiency at which different types of phage are capable of inducing lysis-from-without on different bacteria will vary from phage-to-phage. The concentrations required to induce such a phenomenon may be determined via routine experimentation.

It is described herein that potentially, any surface on which the disinfectant composition can be applied so as to produce high enough concentrations of phage to produce the required lysis-from-without, could be treated. In one aspect the surface is medical equipment, bedding, furniture, walls or floors in a hospital. Alternatively the surface to be treated is preferably the external skin of a mammal, for example the nasal cavity or the surface of a wound or cut in the surface of a mammal. Preferably, the mammal is a human.

The disinfectant composition may be in the form of a spray or liquid wash for the surface. The composition may be a hand wash. Preferably where the composition is a formulation for topical application, it may take the form of a lotion, cream, ointment, paste, gel, foam, or any other physical form as a carrier generally known for topical administration. Such thickened topical formulations are particularly advantageous because the formulations adhere to the area of the skin on which the material is placed, thus allowing a localised high concentration of phage to be introduced to the particular area to be disinfected.

For example, paraffin- and lanolin-based creams, which are particularly useful for the application of product to the nasal cavity, are generally known in the art. However, other thickeners, such as polymer thickeners, may be used.

The formulations may also comprise one or more of the following: water, preservatives, active surfactants, emulsifiers, anti-oxidants, or solvents.

Two or more different phage are used. The surface coatings of different strains of, for example, the same species of bacteria, sometimes varies. Therefore, in order to increase the likelihood that a particular formulation can induce lysis-from-without in the bacterial population of a particular species on a particular surface, it is preferable to use two or more different phage capable of infecting different strains of the same species of bacteria. This also increases the likelihood that lysis-from-within may also be used as a secondary method of killing bacteria on a particular surface.

Two or more different types of phage are used, each phage may be specific for a different species of bacterium. This allows a particular formulation to be used as a control in situations where a number of different bacteria may be present on a particular surface.

Preferably the phage is a pathogen of a bacterium selected from *Staphylococcus, Helicobacter, Klebsiella, Listeria, Mycobacterium*, *Escherichia,* Meningococcus, *Campylobacter*, *Streptococcus, Enterococcus, Shigella*, *Pseudornonas, Burkholderia, Clostridium, Legionella, Acetinobacter*, or *Salmonella.*

Preferably the phage is a pathogen of *Staphylococcus aureus,* especially an MRSA or C. *difficile.*

Indeed, the combination of phage K and phage P68 or mutants thereof is used at both high concentrations as indicated in the claims to induce lysis-from-without for bacterial strains that do not allow multiplication of the phage. The inventor has found that using phage K in combination with phage P68 in a disinfectant formulation allows a broad range of MRSA strains to be infected and killed. The host ranges of phage K and phage P68 complement each other.

A disinfectant composition for disinfecting a surface comprising a carrier, phage K and/or phage P68 or mutants thereof is described herein.

Phage K or phage P68 are used in combination with each other and optionally other phages.

The composition may comprise additional types of phage, in addition to phage K and phage P68.

The mutants of phage K or P68 may be point, deletion or addition mutations. 1, 2, 3, 4, 5, 6, 7, 8, 9 or 10 bases may be changed compared to the original phage K or P68 sequence. Such mutants preferably have an altered host range.

Phage K is discussed in detail in the article by O'Flaherty et al (J. Bacteriol. (2004) 2862-2871). It is a polyvalent phage with a DNA genome of 127,395bp. Typically phage K substantially lacks GATC and GGNCC sites. It typically comprises a large region of the genome having homology to Listeria phage A511. The genome also typically comprises introns in essential phage functions, two in the polymerase gene and one in the lysin gene.

The nucleotide sequence of P68 is shown in the article by Vybiral D. et al (FEMS Microbiol. Lett (2003), 219, 275-283). P68 comprises 18277bp (GenbankNo AF513033).

Preferably the mutation of phage K or phage P68 is at least 90%, most preferably at least 92%, 94%, 96%, 98% or 99% to the native sequence of the phage.

The phage is present in high enough concentrations to induce lysis-from-without as described above.

The composition may be for a topical application, for example, onto the skin of a mammal, such as a human. For example, the skin may be the nasal cavity or on a hand. Other surfaces may be as defined above.

The form of the composition, components of the composition, and uses may be as defined above.

Phage specific for different species of bacteria, and indeed different strains of bacteria, are generally known in the art. A composition comprising one or more generally known phages which are capable of infecting MRSA including phage K and/or phage P68 is described heren.

The invention also provides a disinfectant composition comprising a carrier and two or more phages, the phages comprising phage K or mutants thereof and phage P68 or mutants thereof, characterised in that the concentration of both phages is at least 5:1 pfu phage:cfu bacteria so as to induce lysis-from-without of bacteria for which the phage are a pathogen when said bacteria are present on a surface to be disinfected for use in methods of killing bacteria on a surface comprising applying the disinfectant composition to the surface, wherein the surface is the skin of a mammal, such as a human. In particular, the surface may be the nasal cavity of a mammal, or skin on the hands of a human. This may be used as a disinfectant, for example to prevent the spread of a particular bacterium. It also may be used as a way of inhibiting a bacterial infection on the surface of, for example, skin.

Described herein are methods of treating a bacterial infection comprising applying to an infected surface a composition described herein. There is described a composition herein for use to treat a bacterial infection.

The disinfectant composition may be applied to a bandage or wound dressing.

Described herein is a bandage or wound dressing comprising at least one type of phage, characterised that the phage is provided at a sufficiently high concentration on the bandage or wound dressing to induce lysis-from-without in bacteria for which the phage is a pathogen when contacted with such bacteria.

Described herein is a bandage or wound dressing comprising phage K and/or phage P68 or mutations thereof. Phage K or P68 may be used alone or in combination.

The wound dressing may be a pad or sticking plaster-type dressing. The phage and/or concentrations used are preferably as defined above. The phage may be applied to the wound dressing or bandage as a disinfectant formulation or topical cream, prior to applying to the wound dressing or bandage.

Alternatively, the wound dressing or bandage may be soaked in a carrier containing the phage and dried to leave the phage impregnated within the dressing or bandage.

Phage may also be adsorbed onto the surface of the bandage or wound dressing using techniques generally known in the art.

The advantage of this approach is that the bandage or wound dressing allows the phage to be brought into contact with a wound which may contain the bacteria.

Methods of inhibiting or treating bacteria by applying a bandage or wound dressing to a patient are also described herein.

Bacteriophage K and bacteriophage P68, and phage derived from them, are used in the compositions described herein. These induce lysis-from-without in a wide range of MRSA strains. Both phage are generally known in the art. For example, phage K may be obtained from ATCC (ATCC 19685-B1) and/or P68 from the Felix d'Hérelle Reference Center for Bacterial Viruses from the Université Laval (HER49). Other phage may also be used.

### METHODS

*Staphylococcus aureus* bacteria insensitive to obligately lytic infection by the phage in question were grown in growth medium Luria-Bertani broth to a concentration of approximately 2 x 10⁸ colony forming units (cfu) per ml. Different concentrations of phage were added to different aliquots of suspended bacteria in media and incubated at 37°C overnight.

The turbidity of the culture was measured. A decrease in turbidity indicating lysis-from-without of the cells.

The concentration of phage was calculated as plaque-forming units (pfu) on bacterial cells on which the phage was known to infect and induce lysis-from-within to form plaques. The calculation of pfu of phage and bacterial cells are standard techniques.

Alternatively, a Petri dish of a solid growth medium (Luria-Bertani medium) is provided. Bacteria insensitive to obligately lytic infection by the phage in question are mixed with liquid low density agar and then spread onto the solid growth medium. Aliquots (~ 20 µl) of different dilutions of the phage preparation are then spotted onto the surface of the Petri dish. The Petri dish is incubated to allow the bacteria to grow. Zones of no bacterial growth corresponding to the positions where any of the phage dilutions were spotted indicate lysis from without.

The ability of phage to induce lysis-from-within may be determined by a number of techniques. Typically a petri-dish of a solid growth medium (Luria-Bertani medium) is provided. Bacteria are mixed with phage and liquid low density agar and then spread onto the solid growth medium. The Petridish was incubated to allow the bacteria to grow. Where lysis-from-within occurred, plaques in the bacterial growth were observed.

### RESULTS

Table 1 shows the ability of phage K and phage P68 to induce lysis-from-within (plaques) in a range of different strains of methycillin-resistant *Staphylococcus aureus* (MRSA). SAI 653 has been used as a standard. This is publicly available from the ATCC (ATCC number 19685) and is *Staphylococcus aureus* subsp *aureus* Rosenbach. The remaining strains are MRSA strains isolated from patients at hospitals in the United Kingdom and overseas.

The table also shows that at higher concentrations the phage can be used to induce lysis-from-without in strains including those that the phage would not form plaques on via lysis-from-within.

This considerably increases the effectiveness of formulations comprising phage by increasing the number of different strains of bacteria a formulation containing phage can be used to infect.

It was observed that with phage K, the minimum concentration of phage K needed to induce lysis-from-without on phage in which it was not able to induce plaque formation was 5:1 plaque pfu to cells.

### Lysis from without of Staphylococcus aureus mutants resistant to phage infection.

The *Staphylococcus aureus* phages K, K* and K*710 (spontaneous host range mutants derived from the original parent K) and phage P68 can cause lysis from without of the large majority of *S*. *aureus* strains tested. Experiments were carried out in order to investigate whether *S. aureus* mutants that are resistant to obligately lytic infection (lysis from within) were still sensitive to lysis from without. Five independent mutants of *S. aureus* strain SAI669 (an EMRSA-15 isolate) resistant to phage K*710 were isolated as were another five mutants resistant to phage P68. All of the mutants isolated as being resistant to obligately lytic infection by phage K*710 also exhibited resistance to obligately lytic infection by phages K and K*. Mutants resistant to obligately lytic infection by K/K*/K*710 were sensitive to obligately lytic infection by phage P68 and *vice versa* mutants resistant to obligately lytic infection by phage P68 were still sensitive to obligately lytic infection by K/K*/K*710. Each individual phage-resistant mutant was tested for sensitivity to lysis from without. This test was carried out by inoculating a lawn of the *S. aureus* strain in 3 ml Luria broth top agar (0.7% w/v) onto a plate of Luria broth agar (1.5% w/v) and then spotting 20 µl of 10-fold serial dilutions of each phage (initial concentration -5 x 10⁹ plaque forming units/ml) onto the top agar, followed by incubation at 37 °C for 20 hours. Zones of clearing in the bacterial lawn at high phage concentrations, but the absence of individual plaques at low phage concentrations, were taken to indicate lysis from without.

All ten mutants were sensitive to lysis from without by phages K, K* and K*710. However, mutants resistant to P68 were not sensitive to lysis from without by P68. Spontaneous double mutants of each of the original ten mutants were isolated that were now resistant to both K*710 and P68. These double mutants were screened for their sensitivity to lysis from without. All of the double mutants were sensitive to lysis from without by phages K, K* and K*710, but not by phage P68. From these results it is concluded that mutants of *S. aureus* resistant to obligately lytic infection by phage K and spontaneous host range mutants derived from phage K, remain sensitive to lysis from without.

Thus, within the context of phage therapy, use of phage K and its host range mutant derivatives at sufficiently high concentration should still kill *S. aureus* strains even if they mutate to resistance to obligately lytic infection.

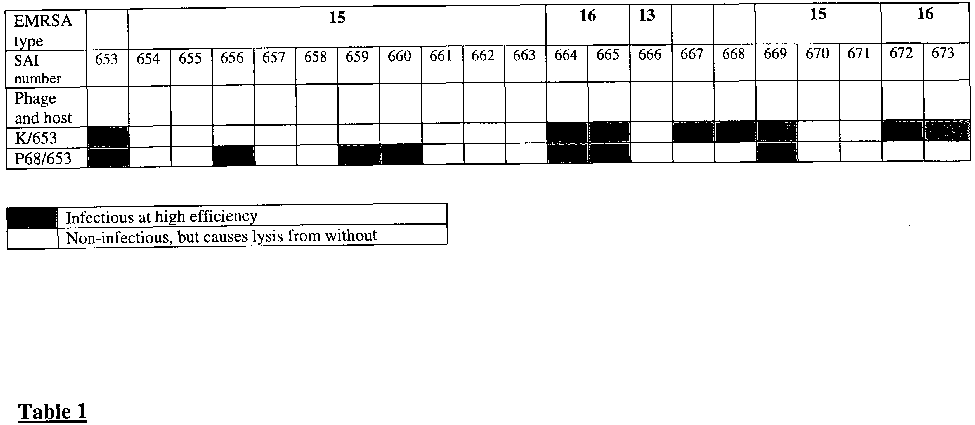

## Claims

1. A method of killing bacteria on a surface being medical equipment, bedding, furniture, walls or floors in a hospital comprising applying to the surface a disinfectant composition comprising a carrier and two or more phages, the phages comprising phage K or mutants thereof and phage P68 or mutants thereof, **characterised in that** the concentration of both phages is at least 5:1 pfu phage:cfu bacteria so as to induce lysis-from-without of bacteria for which the phage are a pathogen when said bacteria are present on the surface to be disinfected.

2. A disinfectant composition comprising a carrier and two or more phages, the phages comprising phage K or mutants thereof and phage P68 or mutants thereof, **characterised in that** the concentration of both phages is at least 5:1 pfu phage:cfu bacteria so as to induce lysis-from-without of bacteria for which the phage are a pathogen when said bacteria are present on a surface to be disinfected for use in a method of killing bacteria on a surface, comprising applying the disinfectant composition to the surface, wherein the surface is the skin of a mammal.

3. A disinfectant composition for use according to claim 2, wherein the disinfectant composition is a topical application for application to the skin of the mammal.

4. A disinfectant composition for use according to claim 3, wherein the skin is within the nasal cavity, or skin of a human's hand.

5. A method according to claim 1, or a disinfectant composition for use according to any one of claims 2 to 4, wherein the disinfectant composition is a spray or liquid wash for the surface.

6. A method according to claim 5, or a disinfectant composition for use according to any one of claims 3 to 5 in the form of a cream, lotion, ointment, paste, gel, foam or hand wash.

7. A method according to claim 5 or 6, or a disinfectant composition for use according to any one of claims 3 to 6, wherein the carrier comprises lanolin or paraffin.

8. A method or disinfectant composition for use according to any preceding claim, wherein the composition comprises further phages which are a pathogen of a bacterium selected from *Staphylococcus, Helicobacter, Klebsiella, Listeria, Mycobacterium, Escherichia, Meningococcus, Campylobacter, Streptococcus, Enterococcus, Shigella, Pseudomonas, Burkholderia, Clostridium, Legionella, Acetinobacter,* or *Salmonella.*

9. A method or disinfectant composition for use according to claim 8, wherein the phage is a pathogen of *Staphylococcus aureus.*

10. A method or disinfectant composition for use according to any preceding claim comprising at least one mutant of phage K or phage P68, wherein the mutant is at least 90%, 92%, 94%, 96%, 98% or 99% identical to the native sequence of the phage.

11. A method or disinfectant composition for use according to claim 10, wherein the mutant is a point, deletion or addition mutant wherein 1-10 bases are changed compared to the original phage K or P68 sequence.

## Patentansprüche

1. Verfahren zum Töten von Bakterien auf einer Oberfläche, die medizinische Vorrichtungen, Bettzeug, Möbel, Wände oder Böden in einem Krankenhaus umfasst, wobei das Verfahren das Aufbringen einer Desinfektionsmittelzusammensetzung auf die Oberfläche umfasst, die einen Träger und zwei oder mehr Phagen umfasst, wobei die Phagen Phage K oder Mutanten davon und Phage P68 oder Mutanten davon umfassen, **dadurch gekennzeichnet, dass** die Konzentration beider Phagen mindestens 5 : 1 pfu-Phagen : cfu-Bakterien beträgt, um die Lyse von außerhalb der Bakterien, für die der Phage ein Pathogen ist, zu induzieren, wenn die Bakterien auf der zu desinfizierenden Oberfläche vorhanden sind.

2. Desinfektionsmittelzusammensetzung, die einen Träger und zwei oder mehrere Phagen umfasst, wobei die Phagen Phage K oder Mutanten davon und Phage P68 oder Mutanten davon umfassen, **dadurch gekennzeichnet, dass** die Konzentration beider Phagen mindestens 5 : 1 pfu-Phagen : cfu-Bakterien beträgt, um die Lyse von außerhalb der Bakterien, für die der Phage ein Pathogen ist, zu induzieren, wenn die Bakterien auf einer zu desinfizierenden Oberfläche vorhanden sind, zur Verwendung in einem Verfahren zum Töten von Bakterien auf einer Oberfläche, das das Auftragen der Desinfektionsmittelzusammensetzung auf die Oberfläche umfasst, wobei die Oberfläche die Haut eines Säugers ist.

3. Desinfektionsmittelzusammensetzung zur Verwendung nach Anspruch 2, wobei die Desinfektionsmittelzusammensetzung eine topische Anwendung zur Aufbringung auf die Haut des Säugers ist.

4. Desinfektionsmittelzusammensetzung zur Verwendung nach Anspruch 3, wobei sich die Haut innerhalb der Nasenhöhle befindet oder die Haut der Hand eines Menschen ist.

5. Verfahren nach Anspruch 1, oder Desinfektionsmittelzusammensetzung zur Verwendung nach Anspruch 2 bis 4, wobei die Desinfektionsmittelzusammensetzung ein Spray oder eine Waschflüssigkeit für die Oberfläche ist.

6. Verfahren nach Anspruch 5 oder Desinfektionsmittelzusammensetzung zur Verwendung nach einem der Ansprüche 3 bis 5 in Form einer Creme, einer Lotion, einer Salbe, einer Paste, eines Gels, eines Schaums oder einer Handwäsche.

7. Verfahren nach Anspruch 5 oder 6, oder Desinfektionsmittelzusammensetzung zur Verwendung nach Anspruch 3 bis 6, wobei der Träger Lanolin oder Paraffin umfasst.

8. Verfahren oder Desinfektionsmittelzusammensetzung zur Verwendung nach einem der vorhergehenden Ansprüche, wobei die Zusammensetzung weitere Phagen umfasst, die ein Pathogen eines Bakteriums sind, das aus Folgendm ausgewählt wird: *Staphylococcus, Helicobacter, Klebsiel*/*a, Listeria, Mycobacterium, Escherichia, Meningococcus, Campylobacter, Streptococcus, Enterococcus, Shigel*/*a, Pseudomonas, Burkholderia, Clostridium, Legionel*/*a, Acetinobacter* oder *Salmonella.*

9. Verfahren oder Desinfektionsmittelzusammensetzung zur Verwendung nach Anspruch 8, wobei der Phage ein Pathogen von *Staphylococcus aureus* ist.

10. Verfahren oder Desinfektionsmittelzusammensetzung zur Verwendung nach einem der vorhergehenden Ansprüche, das bzw. die mindestens eine Mutante des Phagen K oder des Phagen P68 umfasst, wobei die Mutante zu mindestens 90 %, 92 %, 94 %, 96 %, 98 % oder 99 % mit der nativen Sequenz des Phagen identisch ist.

11. Verfahren oder Desinfektionsmittelzusammensetzung zur Verwendung nach Anspruch 10, wobei die Mutante eine Punkt-, Deletions- oder Additionsmutante ist, wobei 1 bis 10 Basen im Vergleich zu dem ursprünglichen Phagen K oder der P68-Sequenz verändert werden.

## Revendications

1. Procédé de destruction de bactéries sur une surface qui est celle d'équipement médical, de literie, de mobilier, de murs ou de planchers dans un hôpital, comprenant l'application sur la surface d'une composition désinfectante comprenant un support et deux phages ou plus, les phages comprenant le phage K ou des mutants de celui-ci et le phage P68 ou des mutants de celui-ci, **caractérisé en ce que** la concentration des deux phages est d'au moins 5:1 ufp de phages:ufc de bactéries de façon à induire une lyse de l'extérieur des bactéries pour lesquelles le phage est un pathogène quand lesdites bactéries sont présentes sur la surface à désinfecter.

2. Composition désinfectante comprenant un support et deux phages ou plus, les phages comprenant le phage K ou des mutants de celui-ci et le phage P68 ou des mutants de celui-ci, **caractérisée en ce que** la concentration des deux phages est d'au moins 5:1 ufp de phages:ufc de bactéries de façon à induire une lyse de l'extérieur des bactéries pour lesquelles le phage est un pathogène quand lesdites bactéries sont présentes sur une surface à désinfecter, destinée à une utilisation dans un procédé de destruction de bactéries sur une surface, la surface étant la peau d'un mammifère.

3. Composition désinfectante destinée à une utilisation selon la revendication 2, la composition désinfectante étant une application topique destinée à une application sur la peau d'un mammifère.

4. Composition désinfectante destinée à une utilisation selon la revendication 3, dans laquelle la peau se trouve dans la cavité nasale, ou est la peau d'une main humaine.

5. Procédé selon la revendication 1, ou composition désinfectante destinée à une utilisation selon l'une quelconque des revendications 2 à 4, la composition désinfectante étant un produit à pulvériser ou un produit de lavage liquide pour la surface.

6. Procédé selon la revendication 5, ou composition désinfectante destinée à une utilisation selon l'une quelconque des revendications 3 à 5, sous la forme d'une crème, d'une lotion, d'une pommade, d'une pâte, d'un gel, d'une mousse, ou d'un produit de lavage des mains.

7. Procédé selon la revendication 5 ou 6, ou composition désinfectante destinée à une utilisation selon l'une quelconque des revendications 3 à 6, dans lequel ou dans laquelle le support comprend de la lanoline ou de la paraffine.

8. Procédé, ou composition désinfectante destinée à une utilisation selon l'une quelconque des revendications précédentes, dans lequel ou dans laquelle la composition comprend des phages supplémentaires qui sont un pathogène d'une bactérie sélectionnée parmi *Staphylococcus, Helicobacter, Klebsiella, Listeria, Mycobacterium, Escherichia, Meningococcus, Campylobacter, Streptococcus, Enterococcus, Shigella, Pseudomonas, Burkholderia, Clostridium, Legionella, Acetinobacter,* ou *Salmonella.*

9. Procédé ou composition désinfectante destinée à une utilisation selon la revendication 8, le phage étant un pathogène de *Staphylococcus aureus.*

10. Procédé, ou composition désinfectante destinée à une utilisation selon l'une quelconque des revendications précédentes, comprenant au moins un mutant du phage K ou du phage P68, le mutant étant au moins 90 %, 92 %, 94 %, 96 %, 98 % ou 99 % identique à la séquence native du phage.

11. Procédé, ou composition désinfectante destinée à une utilisation selon la revendication 10, dans lequel ou dans laquelle le mutant est issu d'une mutation ponctuelle, par délétion ou par addition, dans laquelle 1 à 10 bases sont changées par comparaison avec la séquence originale du phage K ou du phage P68.
